(19) Europäisches Patentamt
European Patent Office
Office européen des brevets

(11) **EP 1 488 774 B1**

(12) **EUROPEAN PATENT SPECIFICATION**

(45) Date of publication and mention
of the grant of the patent:
**14.03.2007 Bulletin 2007/11**

(51) Int Cl.:
***C08F 220/56*** *(2006.01)*

(21) Application number: **04253308.3**

(22) Date of filing: **03.06.2004**

(54) **Polymer compositions and processes for preparing polymers**

Polymerzusammensetzung und Verfahren zur Polymerherstellung

Composition polymère et procédé de fabrication de polymères

(84) Designated Contracting States:
**DE FR GB IT**

(30) Priority: **17.06.2003 US 479079 P**

(43) Date of publication of application:
**22.12.2004 Bulletin 2004/52**

(73) Proprietor: **ROHM AND HAAS COMPANY**
**Philadelphia,**
**Pennsylvania 19106-2399 (US)**

(72) Inventors:
• **Creamer, Marianne Patricia**
**Warrington**
**Pennsylvania 18976 (US)**
• **Greene, Lester William Jr.**
**Willow Grove**
**Pennsylvania 19090 (US)**
• **Kar, Ari Kenneth**
**Warrington**
**Pennsylvania 18976 (US)**
• **Wang, Miao**
**Schwenksville PA 19473 (US)**

(74) Representative: **Kent, Venetia Katherine et al**
**Rohm and Haas (UK) Ltd.**
**European Patent Department**
**4th Floor, 22 Tudor Street**
**London EC4Y 0AY (GB)**

(56) References cited:
**US-A- 4 407 321      US-A- 4 578 267**
**US-B1- 6 569 413**

**Description**

The present invention relates to the use of compositions for treating and modifying hair. More particularly, this invention is directed to styling and conditioning compositions for modifying and fixing hair comprising one or more solution polymers, a process for manufacturing the polymers and a method of using the compositions to modify, condition and fix hair. The invention uses polymers having utility when included in compositions and formulations that are applied to a environment of use, including mammalian skin and hair.

Hair tends to return and recover to its initial shape or position after it is chemically and or physically modified so, as an example, it does not hold a set well. Hair styling and fixative products help create and establish chemically interactive forces between hair fibers, including those which provide, as an example, adherence to the adjacent hairs so they can attain and maintain a particular shape or configuration as the polymer is applied, dries and remains in contact with hair over time. In the past, hairsprays have dominated the styling aid market because of easy use, good styling and simple application. Pump hair sprays, hydrocarbon aerosols and carbon dioxide aerosols are three major types of sprays. However, hairsprays have largely used propellants and alcohol as their major components which are considered Volatile Organic Compounds (VOC).

Government regulations are regularly requiring lower permissible levels of VOC, and chemical industry has responded by reducing the VOC of their products. In most cases, this results in an increase in water content of the formula. But the increase in water content creates many problems such as resin solubility, increased viscosity, loss of holding power, increased initial curl droop and tackiness. In addition, increasing the water content of hair spray can also cause can corrosion and solvent/propellant incompatibility in aerosol formulations. Therefore, non-aerosol and water-based styling aid products such as styling gel, glaze, spray foam, styling cream and waxes, and styling lotion have been gradually replacing hairsprays.

Polymers incorporated in hair fixatives do not provide an adequate balance between conflicting requirements of a water resistant/water insensitive character for good curl retention at high levels of moisture and humidity versus hygroscopic/water sensitive character for rapid and complete removal of the polymer from hair when rinsed with water. Most hair fixative polymers were designed to be soluble in alcohol or propellants, and typically such polymers have poor solubility in water. Polymer performance as a fixative, however, is also affected (typically an adverse or detrimental interaction) when water is incorporated into a hair fixative formulation. Examples which illustrate such an imbalance are cationic polymers such as polyquaternium-11 and polyquaternium-4. The cationic polymers are excellent film-forming polymers, but their high substantivities to hair make them difficult to wash out of hair treated with such polymers. Consequently, anionic and non-ionic polymers are most frequently used as hair fixatives. However, such polymers also have disadvantages associated with their use as hair fixatives. Anionic hair fixative polymers, for example, because of their high solubility in water are also considered too hygroscopic and often exhibit poor hair setting properties in high humidity environments.

U. S. Pat. No. 6,569,413 B1 discloses a cosmetically acceptable fixative composition comprising from 0.1 to about 10 weight percent, based on polymer solids, of an anionic polymer, wherein the anionic polymer is composed of from 10 to 80 mole percent of 2-acrylamido-2-methyl-1-propanesulfonic acid or a base addition salt thereof and from 90 to 20 mole percent of one or more anionic or non-ionic monomers and a method of setting hair using the anionic polymer composition. Both the polymer composition and the process of using the polymer to treat hair, attempt to balance the conflicting requirements of water-resistance for good curl retention at high humidity versus water-sensitivity for rapid and complete removal of the polymer from hair when rinsed with water. However, the polymers lose clarity when formulated in hair treatments (*e.g.* including hair fixatives, hair conditioners) and have poor compatibility with certain thickeners including polycarboxylic acids and carbomers, resulting in lowered and/or unstable formulation viscosities. The polymers also have undesirable humidity resistance, undesirable hair modification properties including modifying hair texture to provide the user a raspy rather than silky feeling as to hair texture and polymer flaking issues after deposition on hair.

Inventors use multi-functional polymers for modifying hair. The invented polymers have an excellent balance of water resistance versus water sensitivity, good color stability and good water stability, the polymers are non-flaking after application to hair, polymers are compatible when combined with additives including neutralizers, surfactants and thickeners. The polymers exhibit unexpected clarity, exhibit stable viscosities when combined with one or more additives in formulations and are not dependent either on a particular neutralizing agent or a sequence of combination with additives when the polymers are formulated. The polymers are used to prepare hair formulations that are clear and non-hazy and hair formulations that have stable viscosities over time and are compatible with formulation additives including neutralizers, surfactants and thickeners, including polycarboxylic acids (*e.g.* poly(acrylic acid) pAA) and carbomers (*e.g.* Carbopol™ available from B.F. Goodrich). Inventors discovered that the compatibility problem with certain rheology modifiers, the problem of clarity of the polymers in formulations and the formulation viscosity stability problem, all associated with anionic solution polymers known from the prior art and described above, are solved by successfully including small amounts of one or more acid-containing monomers in solution polymers used in the present invention. Addition of small amounts of one or more acid-containing monomers to the solution polymers unexpectedly improves the clarity of such polymers in formulations. Moreover, addition of small amounts of acid-containing monomers to certain copolymers known in the art also unexpectedly improves the clarity of such polymers in formulations. Polymers used in the invention, including selected copolymers, terpolymers, tetrapolymers and other solution polymers incorporating a plurality of monomers have unique properties which cannot be attained in anionic solution copolymers taught in the prior art, including acrylamide/ 2-acrylamido-2-methyl-1-propanesulfonic acid (AM/ AMPS™, AMPS™ is available from Lubrizol Corp.) and methacrylic acid/2-acrylamido-2-methyl-1-propanesulfonic acid (MAA/AMPS™) copolymers. The inventors provide a process for preparing the polymers and a process for incorporating the solution polymers into compositions and formulations for treating and modifying hair. The invention provides a process for modifying hair using the solution polymers described herein.

Accordingly, the invention uses a multi-functional polymer composition comprising a copolymer including as monomer units: (a) 50 to 89.9 weight percent of acrylamide and (b) 10 to 40 weight percent of 2-acrylamido-2-methyl-1-propanesulfonic acid and salts thereof; wherein between 0.1 to 5 weight percent of one or more acid containing ethylenically unsaturated monomers is incorporated into the copolymer. According to one embodiment, adding one or more acid-containing ethylenically unsaturated monomers provides a clear, non-hazy composition that has a stable viscosity and is compatible with various additives including neutralizers, surfactants and aqueous thickeners.

The invention may use a process for preparing a multi-functional polymer composition comprising the steps of: (a) preparing a copolymer comprising, as polymerized monomer units, (i) 50 to 89.9 weight percent of one or more ethylenically unsaturated monomers and (ii) 10 to 40 weight percent of 2-acrylamido-2-methyl-1-propanesulfonic acid and salts thereof; wherein from 0.1 to 5 weight percent of one or more acid-containing ethylenically unsaturated monomers is incorporated in the copolymer; and (b) neutralizing the polymer with a base, wherein use of an ethoxylated amine neutralizer is optionally omitted.

"Anionic monomer" refers to a monomer as defined herein which possesses a net negative charge above a certain pH value. Representative anionic monomers include base addition salts of acrylic acid, methacrylic acid, itaconic acid, maleic acid 2-acrylamido-2-methyl-1-propanesulfonic acid, sulfopropyl acrylate or methacrylate or other water-soluble forms of these or other polymerizable carboxylic or sulfonic acids, sulphomethylated acrylamide, phosphoethyl (meth)acrylamide, allyl sulphonate, styrene sulfonic acid, sodium vinyl sulphonate, and the like.

"Monomer" refers to any ethylenically unsaturated group, including polyethylenically unsaturated groups of a compound including allylic, vinylic and acrylic groups. The monomer may be anionic, cationic or non-ionic. The term "other" monomers includes additional anionic, cationic, non-ionic and hydrophobic monomers used to prepare polymers used in the invention. The term "hydrophobic" refers to monoethylenically unsaturated monomers which have low water solubility under the conditions of emulsion polymerization, as described in U.S. Patent No. 5,521,266.

"Non-ionic monomer" refers to a monomer as defined herein which is electrically neutral. Representative non-ionic, water-soluble monomers include acrylamide, methacrylamide, N,N-dimethylacrylamide, N,N-diethylacrylamide, N-isopropylacrylamide, N-vinylformamide, N-vinylmethylacetamide, N-vinyl pyrrolidone, hydroxyethyl methacrylate, hydroxyethyl acrylate, hydroxypropyl acrylate, hydroxypropyl methacrylate, t-butylacrylamide, N-methylolacrylamide, alkyl (meth)acrylates such as methyl(meth)acrylate, butyl acrylate and ethylacrylate, vinyl monomers such as ethylene, styrene, divinylbenzene, di-isobutylethylene, vinyl acetate and N-vinyl pyrolidone, and allyl monomers such as allyl(meth) acrylate. "Cationic monomer" refers to a monomer as defined herein which possesses a net positive charge below a certain pH value. Representative cationic, water-soluble monomers include quaternary ammonium salts of amine functionalized monomers such as acrylamide, methacrylamide, N,N-dimethylacrylamide, N,N-diethylacrylamide, N-isopropylacrylamide, N-vinylformamide, N-vinylmethylacetamide, N-vinyl pyrrolidone, t-butylacrylamide, N-methylolacrylamide, tributylammonium ethyl(meth)acrylate TBAEMA, DMAEMA, DMAPMAM, diallyldimethylammonium chloride DADMAC, methylacrylamidopropyltrimethylammonium chloride MAPTAC, acrylamidopropyltrimethylammonium chloride APTAC, N-vinyl pyrolidone, polyquaternium-11 and polyquaternium-4.

The term "acid-containing monomer refers to ethylenically unsaturated monomers containing carboxylic acid, phosphonic acid, phosphinic acid, sulfinic acid and sulfonic acid groups. Suitable examples include (meth)acrylic acid, maleic acid, succinic acid, itaconic acid, vinyl phosphonic acid and vinylsulfonic acid.

As used herein, the term "salts" refers to the ionic salt resulting from reaction of a carboxylic acid, phosphonic and sulfonic group (-C(O)OH, -P(O)OH, -S(O)OH)) with a base. Suitable bases include alkali metal, alkali earth metal, metal and quaternary ammonium hydroxides, carbonates and bicarbonates; ammonia; primary, secondary and tertiary organic amines. Representative alkali, alkaline earth and metal salts include lithium, sodium, potassium, calcium, magnesium and zinc. Suitable amines include methylamine, ethylamine, ethoxylated amines, diethylamine, triethylamine, pyridine, piperdine, ethanolamine, piperazine, aminoethylpropanol, ethanolamine, diethanolamine triethanolamine. The term "salts" also refers to the ionic salt resulting from reaction of an amine (-$NH_2$), including amides (-$CONH_2$) with an acid. Suitable acids include hydrochloric acid, phosphoric acid, phosphonic acids acetic acid, (meth)acrylic acid, citric acid, sulfonic acids and sulfuric acid.

As used herein, the term "copolymer" refers to polymer compositions containing units of two or more different monomers, the term "terpolymer" refers to polymer compositions containing units of three or more different monomers, and the term "tetrapolymer" refers to polymer compositions containing units of four or more different monomers. A plurality of suitable monomer units is usefully employed in accordance with the invention.

As used herein, the following terms have the designated definitions, unless the context clearly indicates otherwise. The term "alkyl (meth)acrylate" refers to either the corresponding acrylate or methacrylate ester; similarly, the term "(meth)acrylic" refers to either acrylic or methacrylic acid and the corresponding derivatives, such as esters or amides. All percentages referred to will be expressed in weight percent (%), based on total weight of polymer or composition involved, unless specified otherwise. The following abbreviations are used herein: g = grams; ppm = parts per million by weight/volume. Unless otherwise specified, ranges listed are to be read as inclusive and combinable and temperatures are in degrees centigrade (°C).

Polymers used in the present invention typically have a weight average molecular weight ($M_w$) for the backbone polymer of 200 to 800,000, including from 2,000 to 300,000 and from 200,000 to 300,000. Weight average molecular weights for the backbone polymer are based on aqueous phase gel permeation chromatography (GPC) analysis using known polymer standards appropriate for the polymer compositions involved; the polymers are subjected to hydrolysis (to the acid form) prior for determination of the backbone polymer molecular weight. Solution polymers having weight average molecular weights less than 100,000 are usefully employed as hair fixatives and applied in the form aerosols.

Polymers usefully employed according to the invention can be prepared by conventional solution polymerization. According to one embodiment of the invention, the polymers are prepared as solution polymers by a solution polymerization process, including those processes disclosed and described in U.S. Patent Nos. 4,401,650; 4,578,267; 4,859,458; and 4,973,409. According to a separate embodiment, the polymers are prepared by optimizing the solution polymerization conditions, including situations where the polymerization kinetics are not favorable and when acid-containing monomers are used in combination with 2-acrylamido-2-methyl-1-propanesulfonic acid and salts thereof.

According to a separate embodiment, it is contemplated emulsion polymers can be prepared according to polymerization processes including those disclosed in U.S. Patent Nos. 4,427,836; 4,469,825; 4,594,363; 4,677,003; 4,920,160; and 4,970,241 and are also prepared, for example, by polymerization techniques disclosed in European Patent Applications EP 0 267 726; EP 0 331 421; EP 0 915 108 and U.S. Patent Nos. 4,910,229; 5,157,084; 5,663,213 and 6,384,104.

The polymers used in the present invention include, as polymerized units, from 0.1 to 5% of one or more other ethylenically unsaturated acid-containing monomers including monoethylenically unsaturated ($C_3$-$C_6$)carboxylic acid monomers. Suitable monoethylenically unsaturated ($C_3$-$C_6$)carboxylic acid monomers include monoethylenically unsaturated monocarboxylic acids and monoethylenically unsaturated dicarboxylic acid monomers. For example, monoethylenically unsaturated monocarboxylic acids include acrylic acid (AA), methacrylic acid (MAA), $\alpha$-ethacrylic acid, $\beta,\beta$-dimethylacrylic acid, vinylacetic acid, allylacetic acid, ethylidineacetic acid, propylidineacetic acid, crotonic acid, and alkali and metal salts thereof. Suitable monoethylenically unsaturated dicarboxylic acid monomers include, for example, maleic acid, maleic anhydride, fumaric acid, itaconic acid, citraconic acid, mesaconic acid, methylenemalonic acid, and alkali and metal salts thereof. According to one embodiment, the monoethylenically unsaturated ($C_3$-$C_6$)carboxylic acid monomers are selected from one or more of acrylic acid and methacrylic acid. Other suitable ethylenically unsaturated acid-containing monomers include 2-methyl-2-propene-1-sulfonic acid, styrene sulfonic acid, vinyl-sulfonic acid, allylsulfonic acid, methallylsulfonic acid, allyloxybenzenesulfonic acid, methallyloxybenzenesulfonic acid, vinyl phosphonic acid and styrene phosphonic acid.

Polymers used in the present invention include, as polymerized units, from 50 to 89.9%, including from 60 to 75%, of one or more copolymerizable ethylenically unsaturated monomers. Suitable copolymerizable monomers include, for example, butadiene, acrylonitrile, ethylene, di-isobutylethylene, 2-ethylhexyl acrylate, butyl acrylate, butyl methacrylate, 2-hydroxyethyl acrylate, 2-hydroxybutyl methacrylate; styrene, vinyltoluene, t-butylstyrene, isopropylstyrene, and p-chlorostyrene; vinyl acetate, vinyl butyrate, vinyl caprolate; acrylonitrile, methacrylonitrile, butadiene, isoprene, vinyl chloride, vinylidene chloride, N-vinylpyrolidone, hydroxyalkyl (meth)acrylates, ($C_1$-$C_{20}$)alkyl (meth)acrylates, poly(alkyleneoxide) di(meth)-acrylates, amides of ethylenically unsaturated ($C_3$-$C_6$)carboxylic acids, amides of ethylenically unsaturated ($C_3$-$C_6$)carboxylic acids that are substituted at the nitrogen by one or two ($C_1$-$C_4$)alkyl groups, acrylamide, methacrylamide, N-methylol (meth)acrylamide, quaternary ammonium salts of acrylamide, (3-acrylamidopropyl)trimethylammonium chloride, (3-methacrylamidopropyl)-trimethylammonium chloride, quaternary ammonium salts of (meth)acrylate esters (such as 2-(N,N,N-trimethylammonium)ethyl (meth)acrylate), 2-(dimethylamino)ethyl (meth)acrylate, N,N-dimethyl-N-methylacryloxyethyl-N-(3-sulfopropyl)-ammonium betaine, N,N-dimethylaminoethyl(meth)acrylate (DMAEMA), MAPTAC, APTAC, TBAM, TBAEMA, DMADMAM, N,N-dimethylamino-N-dimethyl(meth)acrylamide (DMADMAM), and N,N- dimethyl-N-acrylamidopropyl-N-(3-sulfopropyl)-ammonium betaine.

Polymers used in the present invention include, as polymerized units, from 10 to 40% of 2-acrylamido-2-methyl-1-propanesulfonic acid and salts thereof. Additional suitable copolymerizable monomers include, for example, 2-acrylamido-2-methyl-1-propanesulfonic acid and salts thereof, 2-methacrylamido-2-methyl-1-propanesulfonic acid and salts thereof, 3-methacrylamido-2-hydroxypropane-sulfonic acid, 2-hydroxy-3-(2-propenyloxy)propane-sulfonic acid, 3-sulfopropyl acrylate, 3-sulfopropyl methacrylate, sulfomethyl acrylamide, sulfomethyl methacrylamide and their respective metal salts.

Other monomers usefully employed in the solution polymers used in the invention include non-ionic surfactant monomers incorporating long chain alkyl hydrophobic groups in the polymer, for example, such as $(C_8-C_{20})$alkyl (meth) acrylate monomers (for example lauryl methacrylate) and $(C_8-C_{20})$alkoxy(meth)acrylate poly(alkyleneglycol) monomers, one or more non-ionic vinyl surfactant monomers, selected from the group consisting of an acrylic or methacrylic acid ester of a $C_{12}-C_{24}$ alkyl monoether of a polyalkylene glycol having at least 2 oxyalkylene units therein, including those having at least 6 to 70 oxyalkylene units. Also included are the acrylate and methacrylate surfactant esters selected from the group consisting of: alkyl phenoxy poly(ethyleneoxy)ethyl acrylates and methacrylates; alkoxy poly(ethyleneoxy) ethyl acrylates and methacrylates; wherein ethyleneoxy unit is about 6-70. Such monomers may be defined by the general formula $H_2C=C(R)-C(O)-O(CH_2CH_2O)_nR'$ wherein R is H or $CH_3$, the latter being preferred, n is at least 2, and preferably has an average value of at least 6, up to 40 to 60 and even up to 70 to 100 and R' is a hydrophobic group, for example, an alkyl group or an alkyl phenyl group having 12 to 24 carbon atoms or having an average of 12 to 24 or more carbon atoms. Other suitable monomers include vinyl surfactant monomers that are acid esters of certain nonionic surfactant alcohols. Such surfactant esters are known in the art. For example, Junas et al. U.S. Pat. No. 3,652,497 describe the use of alkylphenoxypoly(ethyleneoxy)ethyl acrylates in preparing several other polymeric surfactant thickeners. Dickstein U.S. Pat. No. 4,075,411 describes several processes for preparing such vinyl surfactant esters including the acid catalyzed condensation of commercially available nonionic polyoxyalkylene surfactant alcohols such as alkylphenoxypoly(ethyleneoxy)ethyl alcohol and block-polymeric glycols with acrylic, methacrylic, crotonic, maleic, fumaric, itaconic or aconitic acid. Alternate esterification methods including alcoholysis and transesterification are also described. Other suitable vinyl surfactant esters can be prepared from monoethers of mixed or heteropolymeric ethyleneoxypropyleneoxy-butyleneoxy polyglycols such as described in Patton U.S. Pat. No. 2,786,080. Additional surfactant alcohols which can be esterified for use herein are given in "McCutcheon's Detergents and Emulsifiers" 1973, North American Edition, Allured Publishing Corp., Ridgewood, N.J. 07450.

Optionally, the solution polymers include a small amount (0.01 to 5% by weight) of at least one polyethylenically unsaturated monomer, to function as a cross-linking agent and to provide a polymer having a network structure. One or more polyethylenically unsaturated monomers may be combined with the monomers during the polymerization process or may be added after the polymerization of monomers. Suitable examples include allyl methacrylate (ALMA), ethylene glycol dimethacrylate (EGDMA), butylene glycol dimethacrylate (BGDMA), diallyl phthalate (DAP), methylenebisacrylamide, pentaerythritol di-, tri- and tetra-acrylates, divinyl benzene, polyethylene glycol diacrylates, bisphenol A diacrylates and combinations thereof. Other suitable cross-linking monomers include glycidyl methacrylate GMA, N-methylol acrylamide MOA and 2-(acetoacetoxy)ethyl methacrylate AAEM. Low levels of the polyethylenically unsaturated monomers are preferred, since levels greater than about 5% by weight tend to over cross-link the polymer or provide a polymer network structure such that their effectiveness in the invention markedly decreases.

According to one embodiment of the invention, the polymer is a copolymer composition comprising, as polymerized monomer units: (a) 50 to 89.9 weight percent of one or more ethylenically unsaturated monomers selected from acrylonitrile, ethylene, vinyl acetate, hydroxyalkyl (meth)acrylates, $(C_1-C_{20})$alkyl (meth)acrylates, poly(alkyleneoxide) di (meth)-acrylates, amides of ethylenically unsaturated $(C_3-C_6)$carboxylic acids, amides of ethylenically unsaturated $(C_3-C_6)$carboxylic acids that are substituted at the nitrogen by one or two $(C_1-C_4)$alkyl groups, acrylamide, methacryla-mide, N-methylol (meth)acrylamide, quaternary ammonium salts of acrylamide, (3-acrylamidopropyl)trimethylammonium chloride, (3-methacrylamidopropyl)-trimethylammonium chloride, quaternary ammonium salts of (meth)acrylate esters (such as 2-(N,N,N-trimethylammonium)ethyl (meth)acrylate), 2-(dimethylamino)ethyl (meth)acrylate, N,N-dimethyl-N-methylacryloxyethyl-N-(3-sulfopropyl)-ammonium betaine N,N-dimethylaminoethyl(meth)acrylate (DMAEMA), N,N-dimethylamino-N-dimethyl(meth)acrylamide (DMADMAM), and N,N- dimethyl-N-acrylamidopropyl-N-(3-sulfopropyl)-ammonium betaine and N,N- dimethyl-N-acrylamidopropyl-N-(3-sulfopropyl)-ammonium betaine; and (b) 10 to 40 weight percent of a monomer selected from 2-acrylamido-2-methyl-1-propanesulfonic acid and salts thereof, 2-methacryl-amido-2-methyl-1-propanesulfonic acid and salts thereof, 3-methacrylamido-2-hydroxypropanesulfonic acid and salts thereof, allylsulfonic acid, methallylsulfonic acid, allyloxybenzenesulfonic acid, methallyloxybenzenesulfonic acid, 2-hydroxy-3-(2-propenyloxy)propane-sulfonic acid, 2-methyl-2-propene-1-sulfonic acid, styrene sulfonic acid, vinyl-sulfonic acid, 3-sulfopropyl acrylate, 3-sulfopropyl methacrylate, sulfomethyl acrylamide and sulfomethyl methacrylamide and 2-acrylamido-2-methyl-1-propanesulfonic acid and their respective salts; and incorporating in the copolymer between 0.1 to 5 weight percent of one or more acid containing ethylenically unsaturated monomers selected from one or more mo-noethylenically unsaturated $(C_3-C_6)$carboxylic acid monomers.

The polymers used in the present invention have numerous advantages over polymers described in U. S. Pat. Nos. 6,569,413; 4,859,458; 4,578,267; and 4,401,650. All prior art publications describe AM/AMPS™ (AMPS™ is available from Lubrizol Corp.) copolymers neutralized with basic compounds, including base addition salts and ethoxylated fatty amines. Polymers used in the invention differ significantly as compared with prior art copolymers in terms of their molecular weight distributions, polymer morphology, polymer properties and the process by which they are prepared.

Polymers prepared according to and described in the latter three publications are good hair conditioners, but are poor hair fixatives. Polymers prepared for the invention are different than polymers prepared according to and described in U. S. Patent No. 6,569,413. The compatibility of polymers used in the invention in thickeners including polycarboxylic acids and carbomers such as Carbopol™ is better than comparable anionic copolymers, such as AM/AMPS™ and MAA/AMPS™ copolymers. The present invention uses multi-functional solution polymers that have utility for modifying hair, said polymers having an excellent balance of moisture (water) resistance versus water sensitivity, polymers having good color stability, good water stabilities, polymers that are non-flaking after application, and polymers that are compatible with thickeners including polyacrylic acids, paolycarboxylic acids and carbomers such as Carbopol™, providing clear, non-hazy hair formulations having stable formulation viscosities over time. Moreover, the formulations provide a smooth coating on hair, provide better adhesion to hair follicles and skin surfaces, and improve the sheen and glossiness of hair treated with the polymers used in the invention. Inventors discovered that the formulation compatibility, clarity and viscosity stability problems associated with anionic emulsion polymers of the prior art are solved utilizing critical amounts of one or more acid-containing monomers in the polymer. The polymers used in the invention are used to prepare hair formulations that are clear, non-hazy hair formulations having stable viscosities over time and are compatible with formulation additives including neutralizers, surfactants and thickeners such as polycarboxylic acids and carbomers including Carbopol™ for example. Inventors discovered that the compatibility problem with certain rheology modifiers and the viscosity stability problem, both associated with anionic solution polymers known from the prior art and described above, is unexpectedly solved by successfully including small amounts of one or more acid monomers in solution polymers used in the present invention.

Solution polymers used in the present invention are different as compared to the anionic solution AMPS™ homopolymers and copolymers AM/AMPS™ and MAA/AMPS™. The AMPS™ monomer and salts thereof is critical for hair conditioning, but alone does not achieve the required balance of conditioning effect versus hair modification effect. Moreover, AM/AMPS™ and MAA/AMPS™ solution copolymers lose clarity when formulated in hair treatments (*e.g.* including hair fixatives, hair conditioners) and have poor compatibility with certain thickeners including polycarboxylic acids and carbomers, resulting in lowered and/or unstable formulation viscosities. The polymers also have undesirable humidity resistance, undesirable hair modification properties including modifying hair texture to provide the user a raspy rather than silky feeling as to hair texture and polymer flaking issues after deposition on hair. Addition of small amounts of one or more acid-containing monomers to the solution polymers used in the invention unexpectedly improves the clarity of such polymers in formulations. Moreover, addition of small amounts of acid-containing monomers to certain copolymers known in the art also unexpectedly improves the clarity of such polymers in formulations. The solution polymers used in the present invention have no such limitations and achieve the necessary balances of properties and effects when applied to mammalian skin and hair. Acid-containing ethylenically unsaturated monomers (acem) do not react well with AMPS™, and typically result in solution polymers having high levels of residual monomer after polymerization. However it was discovered that, when small amounts of such monomers are added to AM/AMPS copolymers, an unexpected improved clarity and stable viscosity is observed. The solution polymers used in the invention, AMPS™/acem/AM and AMPS™/acem, further provide improved polymer clarity and stable polymer viscosities as compared to the anionic solution copolymers AM/AMPS™ and MAA/AMPS™ and AMPS™ homopolymer. Moreover, the resulting polymers used in the present invention have different and unique polymer properties as compared to the anionic solution copolymers AM/AMPS™ and MAA/AMPS™. The inventors also discovered that AM copolymers and copolymers incorporating AM are markedly unique as compared with respective copolymers incorporating AA and MAA with AMPS™ monomers. AM has a better initiation profile, better polymerization kinetics and the polymerization process provides polymers with comparatively lower residual monomer levels. The process of preparing solution polymers used in the invention provides a significant improvement in terms of cycle time, safety, consistency of operation, quality control, and polymer performance as compared to solution polymers known in the prior art.

According to one embodiment of the present invention, the solution polymers are prepared by conventional solution polymerization techniques known in the art. For example, these polymers can be prepared by polymerization of monomers dissolved in an aqueous solvent. Both batch and continuous processes can be used.

According to a separate embodiment, among batch processes for preparing the polymers, a unexpectedly effective process was discovered that combines both single/multiple shot and gradual addition processes also referred to as a hybrid gradual addition/shot addition process. The process used for the invention provides a significant improvement in terms of cycle time, safety, consistency of operation, quality control, and polymer performance.

Optionally, the polymers used in the present invention may also be made using known techniques, for example, solution (aqueous or solvent), emulsion, solvent-exchange (solution polymerization followed by phase inversion) or suspension polymerization; the polymerizations can be conducted as co-feed, heel, semi-continuous or continuous processes. The polymers may be random or block polymers depending upon the specific method used to conduct the polymerization. The polymers may be used in solution form, for example as aqueous solutions, or they may be isolated as solid materials, for example by drying, including for example spray drying, and used in the form of granules or particulates.

Conventional means for initiating the polymerization of ethylenically unsaturated monomers, including both thermal and redox initiation systems, is used. Initiators useful for these polymerizations are any of the well known free-radical-producing compounds such as peroxy, hydroperoxy and azo initiators. The polymerization of monomers is performed in a suitable solvent and in the presence of an initiator. Suitable solvents include for example water, dioxane, ketones such as 4-methylbutan-2-one, aromatic hydrocarbons such as toluene, xylene and xylene isomers, alcohols such as methanol, isopropanol and ethanol and ethers such as dioxane. Suitable reaction initiators include for example azo(bis)isobutyronitrile (AIBN), organic peroxides such as benzoyl peroxide, di-t-butyl peroxide, hydroperoxides such as t-butyl hydroperoxide and t-amyl hydroperoxide, hydrogen peroxide, sodium perborate, alkali metal persulfates and ammonium persulfate. The initiator concentration is normally between 0.01 and 6% by weight based on the total weight of the monomers, including from 0.1 to 4%. Chain transfer agents may also be added to the polymerization reaction to control the molecular weight of the polymer. Suitable chain transfer agents include alkyl mercaptans such as lauryl (dodecyl) mercaptan, carbon tetrachloride, bromoform, bromotrichloromethane, long chain alkyl mercaptans and thioesters such as n-dodecyl mercaptan, t-dodecyl mercaptan, octyl mercaptan, tetradecyl mercaptan, hexadecyl mercaptan, butyl thioglycolate, isooctyl thioglycolate, and dodecyl thioglycolate; bisulfites, phosphorous acid and salts thereof; hypophosphite salts such as sodium hypophosphite; and metal salts of Fe and Cu. The chain transfer agents are used in amounts up to 10 parts per 100 parts of polymerizable monomers. The concentration of chain transfer agent used is from 0 to about 1.0% by weight.

Water-soluble redox initiators are also used. Redox initiators include, for example, sodium bisulfite, sodium sulfite, hypophosphites, phosphites, isoascorbic acid, sodium formaldehyde-sulfoxylate and hydroxylamines, used in conjunction with suitable oxidizing agents, such as the thermal free-radical initiators noted above. The redox initiators are typically used in amounts from 0.05 to 10%, preferably from 0.5 to 5%, based on the weight of total monomer. Combinations of initiators can also be used.

Suitable oxidants of the redox initiator system include water-soluble oxidizing compounds such as, for example, hydrogen peroxide, peroxy acid salts, peroxodisulfuric acid and its salts, peroxy ester salts, ammonium and alkali metal peroxide salts, perborate salts and persulfate salts. Suitable oxidants of a redox initiator system also include water-insoluble oxidizing compounds such as, for example, dibenzoyl peroxide, t-butyl peroxide, lauryl peroxide, 2, 2'-azo bis (isobutyronitrile) (AIBN), alkyl hydroperoxides such as tert-butyl hydroperoxide, tert-amyl hydroperoxide, pinene hydroperoxide and cumyl hydroperoxide, t-butyl peroxyneodecanoate, and t-butyl peroxypivalate. Compounds which donate oxygen with free radical formation and are not peroxides, such as alkali metal chlorates and perchlorates, transition metal oxide compounds such as potassium permanganate, managanese dioxide and lead oxide and organic compounds such as iodobenzene, may be usefully employed in accordance with the invention as oxidants. The term "water-insoluble" oxidants means oxidizing compounds having a water solubility of less than 20 % by weight in water at 25° C. Typical levels of oxidant range from 0.01% to 3.0%, including from 0.02% to 1.0% and from 0.05% to 0.5% by weight, based on the weight of the monomer used.

Suitable reductants of the redox initiator system include reducing compounds such as, for example, sulfur compounds with a low oxidation state such as sulfites, hydrogen sulfites, alkali metal bisulfites, ketone adducts of bisulfites such as acetone bisulfite, alkali metal disulfites, metabisulfites and its salts, thiosulfates, formaldehyde sulfoxylates and its salts, reducing nitrogen compounds such as hydroxylamine, hydroxylamine hydrosulfate and hydroxylammonium salts, polyamines and reducing sugars such as sorbose, fructose, glucose, lactose and derivatives thereof, enediols such as ascorbic acid and isoascorbic acid, sulfinic acids, hydroxy alkyl sulfinic acids such as hydroxy methyl sulfinic acid and 2-hydroxy-2-sulfinacetic acid and its salts, formadinesulfinic acid and its salts, alkyl sulfinic acids such propyl sulfinic acid and isopropyl sulfinic acid, aryl sulfinic acids such as phenyl sulfinic acid. The term "salts" includes for example sodium, potassium, ammonium and zinc ions Typical levels of reductant range from 0.01% to 3.0%, including from 0.01% to 0.5% and from 0.025% to 0.25% by weight, based on the weight of the monomer used.

The metal promoter complex of the redox initiator system includes a water-soluble catalytic metal compound in the form of a salt and a chelating ligand. Suitable metal compounds include metal salts such as, for example iron(II, III) salts such as iron sulfate, iron nitrate, iron acetate and iron chloride, cobalt(II) salts, copper(I, II) salts, chromium (II) salts, manganese salts, nickel(II) salts, vanadium salts such as vanadium(III) chloride, vanadium(IV) sulfate and vanadium (V) chloride, molybdenum salts, rhodium salts and cerium(IV) salts. It is preferred that metal compounds are in the form of hydrated metal salts. Typical levels of catalytic metal salts used in accordance with the invention range from 0.01 ppm to 25 ppm. Mixtures of two or more catalytic metal salts may also be usefully employed in accordance with the invention.

Metal complexes that promote the redox cycle in a redox initiator system must not only be soluble, but must have suitable oxidation and reduction potentials. Generally stated, the oxidant must be able to oxidize the low oxidation state of metal promoter complex (e.g. Fe(II)-> Fe(III)) and conversely, the reductant must be able to reduce the high oxidation state of the metal promoter catalyst (e.g. Fe(III)-> Fe(II)). The choice of a particular oxidant and reductant usefully employed in a redox initiator system for preparing aqueous emulsion polymers from two or more ethylenically unsaturated monomers depends on the redox potentials of the metal salts. In addition, the ratio of oxidant to reductant ranges from 0.1:1.0 to 1.0:0.1, depending on the redox potential of the metal salt employed. For the efficient reduction of monomer levels in an aqueous polymer dispersion prepared from one or more ethylenically unsaturated monomers, it is preferred that the chelating ligand used in combination with the soluble metal salt is a multidentate aminocarboxylate ligand having fewer than six groups available for coordination to the metal salt.

Oxidant and reductant are typically added to the reaction mixture in separate streams or as a single shot, preferably concurrently with the monomer mixture. The reaction temperature is maintained at a temperature lower than 100 °C throughout the course of the reaction. Preferred is a reaction temperature between 30 °C and 85 °C, preferably below 60°C. The monomer mixture may be added neat or as an emulsion in water. The monomer mixture may be added in one or more additions or continuously, linearly or not, over the reaction period , or combinations thereof. The type and amount of redox initiator systems may be the same or different in the various stages of the emulsion polymerization.

Optionally, an anionic emulsifier is included in the polymerization charge and one or more of the known nonionic emulsifiers may also be present. Examples of anionic emulsifiers are the alkali metal alkyl aryl sulfonates, the alkali metal alkyl sulfates and the sulfonated alkyl esters. Specific examples of these well-known emulsifiers are sodium dodecylbenzenesulfonate, sodium disecondary-butylnaphthalene sulfonate, sodium lauryl sulfate, disodium dodecyldiphenyl ether disulfonate, disodium n-octadecylsulfosuccinamate and sodium dioctylsulfosuccinate.

Polymerization processes for the preparation of polymers used in the present invention generally result in good conversion of the monomers into polymer product. However, if residual monomer levels in the polymer mixture are undesirably high for a particular application, their levels can be reduced by any of several techniques. One common method for reducing the level of residual monomer in a polymer mixture is the post-polymerization addition of one or more initiators or reducing agents to assist scavenging of unreacted monomer.

Preferably, any post-polymerization additions of initiators or reducing agents are conducted at or below the polymerization temperature. The initiators and reducing agents suitable for reducing the residual monomer content are well known to those skilled in the art. Generally, any of the initiators suitable for the polymerization are also suitable for reducing the residual monomer content of the polymer mixture. The level of initiators or reducing agents added as a means for reducing the residual monomer content should be as low as possible to minimize contamination of the product. Generally, the level of initiator or reducing agent added to reduce the residual monomer content is in the range from 0.01 to 2.0 mole %, preferably from 0.5 to 1.0 mole %, based on the total amount (moles) of polymerizable monomer.

Further general and specific details on preparation of polymers used in the present invention by solution polymerization followed by phase inversion may be found in Progress in Organic Coatings, **29**, p 211 (1996) and Progress in Organic Coatings, 26, p 207 (1995).

The glass transition temperature (Tg) of polymers usefully employed in accordance with the invention are of a wide range and will vary according to the polymer morphology of a particular solution polymer composition. The glass transition temperature ("Tg") of the polymers used herein are those calculated by using the Fox equation (T.G. Fox, Bull. Am. Physics Soc., Volume 1, Issue No. 3, page 123(1956)). that is, for calculating the Tg of a copolymer of monomers M1 and M2,

$$1/Tg(calc.) = w(M1)/Tg(M1) + w(M2)/Tg(M2)$$
,

wherein

Tg(calc.) is the glass transition temperature calculated for the copolymer
w(M1) is the weight fraction of monomer M1 in the copolymer
w(M2) is the weight fraction of monomer M2 in the copolymer
Tg(M1) is the glass transition temperature of the homopolymer of M1
Tg(M2) is the glass transition temperature of the homopolymer of M2,
all temperatures being in °K.

The glass transition temperatures of homopolymers may be found, for example, in "Polymer Handbook", edited by J. Brandrup and E.H. Immergut, Interscience Publishers.

As used herein, the term "water soluble", as applied to monomers and polymers, indicates that both the monomer and resulting polymer has a solubility of at least 1 gram per 100 grams of water, preferably at least 10 grams per 100 grams of water and more preferably at least about 50 grams per 100 grams of water. The term "water insoluble", as applied to monomers and polymers, refers to monoethylenically unsaturated monomers and the resulting polymers which have low or very low water solubility under the conditions of polymerization, as described in U.S. Patent No. 5,521,266. An aqueous system refers to any solution containing water.

Solution polymers used in the invention are used as compositions for treating hair by incorporating them in a cosmetically acceptable medium in amounts of from 0.1 to about 10 weight percent, including from 0.5 to about 5 percent by weight, based on total polymer solids.

The polymers used in the invention can be incorporated as compositions and formulations in various forms including hair spray, styling gel, styling glaze, spray foam, styling cream, styling wax, styling lotion, liquid foam and mousse. They can contain water and also any cosmetically acceptable solvent, in particular monoalcohols, such as alkanols having 1 to 8 carbon atoms, like ethanol, isopropanol, benzyl alcohol and phenylethyl alcohol, polyalcohols, such as alkylene glycols, like glycerins, ethylene glycol and propylene glycol, and glycol ethers, such as mono-, di- and tri-ethylene glycol monoalkyl ethers, for example ethylene glycol monomethyl ether, ethylene glycol monomethyl ether and diethylene glycol monomethyl ether, used singly or in a mixture. These solvents can be present in proportions of up to as much as 99.5 percent by weight, relative to the weight of the total composition.

The polymers used in the invention are compatible in the one or more additives, including thickeners, rheology modifiers, other hair fixative polymers, other polymers, neutralizers, humectants, surfactants, conditioning agents, silicones, colors, dyes, fragrances, naturally occurring materials and preservatives; and can be neutralized at any stage of preparing the formulation. Suitable naturally occurring materials include soy bean oil, cellulose, modified cellulose, castor oil and linseed oil. In addition, the compositions and formulations incorporating polymers used in the invention also can contain any other ingredient normally used in cosmetics, such as perfumes, dyestuffs (also referred to as coloring agents and colorants) which can serve to color the composition itself or the fibers (also referred to as follicles) of the hair, preservatives, sequestering agents, thickeners, silicones, softeners, foam synergistic agents, foam stabilizers, sun filters, peptizing agents and also anionic, non-ionic, cationic or amphoteric surface-active agents or mixtures thereof.

Hair fixative compositions and formulations comprising polymers used in the invention are applied to wet or dry hair by spraying or by rubbing onto the hair manually. The treated hair is then mechanically fixed in the desired configuration using, for example, any of a variety of rollers or curlers. In the case of application to wet hair, the hair is then dried using ambient air, electric or hot air drying using, for example, a blow dryer. The hair is then combed to provide the desired hairstyle. After use, the hair is rinsed with water to remove the hair fixative.

According to a separate embodiment of the invention, hair fixative compositions and formulations incorporating the polymers used in the invention are selected from the group consisting of gels, glazes and creams.

A hair styling gel is firm gel that thins upon application of shear such that it spreads very thin when applied to hair. Hair styling gels are typically applied by manually rubbing the gel onto wet or damp hair. The hair is then placed in the desired configuration, for example by wrapping the hair tightly around curlers or a finger and set by drying as described above. For a general treatise of hair styling and setting, see C. Zviak, The Science of Hair Care, 150-178 (1986).

Hair styling glazes are easy to spread, clear flowable gels that are particularly useful for the wet look or blow dry styling methods. Hair styling creams are easy to spread, flowable lotions.

In addition to the polymer used in the invention and water and/or alcohol, the hair styling gel or glaze contains about 0.05 to about 15 percent by weight of a thickener. The thickener should be compatible with the anionic polymer and should not adversely affect the stability or efficacy of the hair styling gel. Representative thickeners include poly(meth)acrylic acids (available from Rohm and Haas Company, Philadelphia, PA under the tradename Acumer™), carbomers which refer to polyacrylic acid cross-linked with allyl ethers of pentaerythrol or allyl ethers of sucrose (available from BF Goodrich, Brecksville, Ohio under the tradename Carbopol™), sodium acrylates copolymer (available from Ciba Specialty Chemicals Corporation, High Point, N.C. under the tradename Salcare™), xanthan gums, sodium alginates, gum arabic and cellulose derivatives. It is also possible to achieve thickening by means of a mixture of polyethylene glycol stearates or distearates or by means of a mixture of a phosphoric acid ester and an amide.

Other optional ingredients are also incorporated into the hair styling gel or glaze. The identity of the optional ingredients is not limited as long as the optional ingredients do not adversely affect the aesthetics or efficacy of the hair styling gel. Such optional ingredients are well known to those skilled in the art and include emulsifiers such as anionic or nonionic surfactants; preservatives such as benzyl alcohol, methyl paraben, propyl paraben, or imidazolidinylurea; cationic conditioners such as cetyl trimethyl ammonium chloride, methyldibromoglutaronitrile (available from ONDEO Nalco, Naperville, Ill. under the tradename Merguard™), stearyl dimethyl benzyl ammonium chloride, isothiazolones such as Kathon™ and Neolone™ (available from Rohm and Haas Company, Philadelphia, PA), and di(partially hydrogenated tallow) dimethyl ammonium chloride; coloring agents such as any of the FD&C or D&C dyes; perfume oils; and chelating agents such as ethylenediaminetetraacetic acid.

Hair fixatives incorporating polymers used in the invention may also contain conventional hair care adjuvants including plasticizers such as glycols, phthalate esters and glycerine, silicones, emollients, lubricants, and penetrating agents such as various lanolin compounds, protein hydrolysates and other protein derivatives, ethylene adducts and polyoxyethylene cholesterol.

The hair fixative incorporating polymers used in the invention can also contain electrolytes, such as aluminum chlorohydrate, alkali metal salts, e.g., sodium, potassium or lithium salts, these salts preferably being halides, such as the chloride or bromide, and the sulphate, or salts with organic acids, such as the acetates or lactates, and also alkaline earth metal salts, preferably the carbonates, silicates, nitrates, acetates, gluconates, pantothenates and lactates of calcium, magnesium and strontium. Other suitable electrolytes are metal cross-linking agents including salts of magnesium, calcium and zinc. The metal salts are also suitable cross-linking agents used in preparing cross-linked solution polymers used in the invention.

The hair fixatives prepared using polymers used in the invention may also contain one or more additional hair fixative polymers. When present, the additional hair fixative polymers are present in a total amount of from about 0.25 to about 5 percent by weight. Representative hair fixative polymers compatible with anionic and nonionic hair fixative polymers include acrylic/acrylate copolymers, allyl stearate/vinyl acetate (VA) copolymers, AMP acrylate/diacetoneacrylamide copolymers, butyl ester of ethylene/maleic anhydride (MA) copolymers, butyl ester of PVM/MA copolymers, acrylate/C1-20 succinate/hydroxyacrylate copolymers, including Allianz™ LT-120 (available from Rohm and Haas Company, Philadelphia, PA and ISP, Wayne, New Jersey), acrylate/hydroxyester acrylates, including Acudyne™ 180 (available from Rohm and Haas Company, Philadelphia, PA), isopropyl ester of PVM/MA copolymers, octylacrylamide/acrylate/ butylaminoethyl methacrylate copolymers, phthalic anhydride/glycerin/glycidyl decanoate copolymers, polybutylene terephthalate PBT, polyethylacrylate, polyethylene, polyvinyl acetate, polyvinyl butyral, polyvinyl methyl ether, polyvinylprrolidinone (PVP), poly N-vinylformamide, PVP/VA copolymers, PVP/dimethylaminoethylmethacrylate copolymers, PVP/eicosene copolymers, PVP/ethyl methacrylate/methacrylic acid copolymers, PVP/hexadecene copolymers, PVP/VA/itaconic acid copolymers, sodium acrylate/vinyl alcohol copolymers, starch diethylaminoethyl ether, stearylvinyl ether/maleic anhydride copolymers, VA/crotonate copolymers, VA/crotonic acid copolymers, VA/crotonic acid/methacryloxybenzophenone-1 copolymers, VA/crotonic acid/vinyl neodecanoate copolymers, and combinations thereof.

Hair styling gels using polymers used in the present invention are prepared by dissolving the polymers used in the invention in water or a water/alcohol mixture, with heating if necessary. An aqueous solution of the viscosity enhancer and any optional ingredients are then added and the mixture is stirred to provide the gel or glaze.

When the hair fixative incorporating polymers used in the invention is in the form of a hair spray or mousse, it additionally contains up to 50 weight percent of one or more propellants. Typical propellants include ethers, compressed gases, halogenated hydrocarbons and hydrocarbons including, dimethyl ether, carbon dioxide, nitrogen, nitrous oxide and volatile hydrocarbons, such as butane, isobutane, propane, and the like.

Polymers used in the invention are easily incorporated into other useful compositions and formulations, including but not limited to, gels, setting agents, setting creams, pomade, waxing agents, oil treatments, foams, mousses, gels that can be sprayed, shine agents, conditioners left on skin and hair, conditioning agents, softeners, rinse off conditioners, shampoos, shampoos including conditioners, hair color treatments, hair bleaching treatments, agents for increasing hair volume, moisturizers, soaps, cosmetics, body washes, shaving preparations (*e.g.* lotions, creams, gels and glazes), sunscreens, topical skin and eye treatments.

The polymers used in the invention are usefully employed for cosmetic purposes as film formers without requiring addition of other materials, for example, such as a hair fixative. The polymers can also be formulated with other ingredients known to the cosmetic industry and registered under CTFA International Cosmetic Ingredients Dictionary and Handbook. Such ingredients include emollients, humectants, other film forming polymers, propellants, solvents, pigments, dyes, buffers, organic and inorganic suspending and thickening agents, waxes, surfactants and co-surfactants, plasticizers, organic and inorganic neutralizing agent, preservatives, flavoring agents, perfumes, and active ingredients including sunscreen agents, insect repellents, vitamins, herbal extracts, antiperspirant and deodorant agents, skin or hair bleaching or coloring agents, depilating agents, anti-fungal and antimicrobial agents, anti-dandruff and anti-acne agents, astringents, and combinations thereof.

Cationic surfactants are also usefully employed as additives in compositions and formulations used in the invention. Cationic surfactants contain hydrophilic functional groups and include octylbenzyltrimethylammonium chloride, hexadecyltrimethylammonium chloride, hexadecyltrimethylammonium bromide and dodecyltrimethylammonium chloride), oxygen containing amines, quaternary amine salts, ester groups containing quaternary ammonium salts, as disclosed in European patent Publication Nos. EP 0 345 842 A2, EP 0 239 910 and U.S. Pat. No. 4,137,180. Other suitable cationic surfactants include methylbis (tallowamidoethyl)(2- hydroxyethyl) ammonium methyl sulphate, methyl bis (hydrogenated tallowamido ethyl)(2 hydroxyethyl) ammonium methyl sulphate, imidazolinium salts, including 1-methyl-1-(tallowylamido) ethyl-2-tallowyl-4,5 dihydro imidazolinium methosulphate and 1-methyl-1-(palmitoylamido) ethyl-2-octadecyl-4,5-dihydroimidazolinium chloride.

Non-ionic surfactants are also usefully employed as additives in the invention. In one embodiment they are additives. Non-ionic surfactants are surfactants having no charge when dissolved or dispersed in aqueous solutions. Typical nonionic surfactants useful in the present invention include, for example, $(C_6-C_{18})$alkylphenol alkoxylates (such as t-octyl phenol and nonylphenol ethoxylates having 1-70, and preferably 5-16, ethyleneoxide units), $(C_{12}-C_{20})$alkanol alkoxylates and block copolymers of ethylene oxide and propylene oxide; optionally, the end groups of polyalkylene oxides can be blocked, whereby the free OH groups of the polyalkylene oxides can be etherified, esterified, acetalized and/or aminated. Another modification consists of reacting the free OH groups of the polyalkylene oxides with isocyanates. Useful non-ionic surfactants also include, for example, $(C_4-C_{18})$alkyl glucosides as well as the alkoxylated products obtainable therefrom by alkoxylation, particularly those obtainable by reaction of alkyl glucosides with ethylene oxide.

Amphoteric or zwitterionic surfactants (such as cocamidopropyl betaine) including both acidic and basic hydrophilic groups and can also be used as additives in the present invention.

Optionally, anionic surfactants are used as additives. Anionic surfactants are surfactants having a hydrophilic functional group in a negatively charged state in an aqueous solution. Typical anionic surfactants include, for example, $(C_8-C_{18})$alkyl carboxylic acids, $(C_{12}-C_{20})$sulfonic acids (sulfonated alkylaryl compounds such as sodium dodecylbenzenesulfonate), $(C_{10}-C_{20})$sulfuric acid esters (sulfated alcohols such as lauryl and cetyl sulfates, sodium salts), phosphate esters and salts thereof.

In one embodiment, non-ionic surfactants, such as alcohol ethoxylates are usefully employed as additives in the present invention. However, mixtures of non-ionic surfactants with anionic surfactants, non-ionic surfactants with cationic surfactants, non-ionic surfactants with amphoteric surfactants, anionic surfactants with amphoteric surfactants, and cationic surfactants with amphoteric surfactants may be used as long as they are compatible and satisfy the balance of hydrophilic-lipophilic (HLB) properties described below.

As used herein, HLB is a value characterizing the relative proportions of hydrophilic and lipophilic (also referred to as hydrophobic) portions of molecules, such as the polyetherurethane associative thickeners and the selected surfactants of the present invention; higher HLB values (those approaching 50) represent the more hydrophilic molecules and the lower HLB values (those around 6 to 10) represent the more hydrophobic molecules. HLB values may be calculated or determined by a variety of known procedures, such as those described in "Surfactants and Interfacial Phenomena" by Milton J. Rosen, John Wiley and Son, New York, NY, page 244 (1978) and "Interfacial Phenomena" by J.T. Davies and E.K. Rideal, Academic Press, 2nd Edition, pp 373-383 (1963).

The HLB range of the one or more surfactants usefully employed in accordance with the invention will vary depending on the nature of the polymer. The HLB range usefully employed for most aqueous emulsion polymers is between 10 and 25, depending on the hydrophilic/hydrophobic character of monomers used to prepare a specific solution polymer and the water solubility/insolubility of the resulting polymer.

The solution polymers used in the invention are processed into solids by conventional techniques including but not limited to freeze drying, evaporation, evaporation under reduced pressure, spray drying, fluidized spray drying and coagulation using cationic surfactants, polyelectrolytes, metal salts or combinations thereof. The drying/isolation technique usefully employed according to the invention will vary depending on the nature of the aqueous emulsion polymer, the surfactant(s) utilized and combinations thereof.

The invention may use a manufacturing process for preparing solution polymers that have desired rheology and polymer properties for incorporating into compositions and formulations used in treating and modifying hair. Compositions and formulations incorporating the polymers prepared by this method may be applied to skin and hair at coating volumes, for example, of from about 0.5 microliters per square centimeter ($\mu$L/cm$^2$) to about 4 $\mu$L/cm$^2$.

Some embodiments of the invention are described in detail in the following Examples. All ratios, parts and percentages are expressed by weight unless otherwise specified, and all reagents used are of good commercial quality unless otherwise specified. The following abbreviations are used in the Examples:

AM =      Acrylamide
AMPS =     2-acrylamido-2-methyl-1-propanesulfonic acid, sodium salt
MAA =     Methacrylic Acid
AA =      Acrylic Acid

Brookfield viscosities of polymers used in the invention and of hair compositions and hair formulations incorporating polymers used in the invention were measured using a commercially available Brookfield viscometer. Details of the Brookfield viscosity measurements, and interpretation of Brookfield values are described by Christopher W. Macosko in "Rheology: Principles, Measurements and Applications, VCH Publishers: New York, 1994.

**Example 1 (75AM/25 AMPS™). Comparative Example 1**

The 75AM/25AMPS™ copolymer was prepared according to the method described in U.S. Patent Nos. 4,578,267 as a comparative example.

A one liter resin kettle with overhead stirrer, $N_2$ inlet, condenser, thermocouple, heating mantle, and provision for external cooling was set up in a hood. 24.5 g of AMPS™ (0.118 m) was dissolved in 118 ml of 1N NaOH and the pH adjusted to 8, the total weight was 159.3 g. This solution was then added to the kettle along with 152.8 g of 49.4% Dow aqueous acrylamide (1.06 m) and 100 ml of $H_2O$. Then 0.038 g of $CuCl_2 \cdot 2H_2O$ dissolved in 62 ml of $H_2O$ was added. Heating, stirring, and $N_2$ purging was performed. After about 40 minutes when, after reaching a temperature of 50°C, the heating mantle was removed and 0.50 g of $(NH_4)_2 S_2O_8$ dissolved in 25 ml of $H_2O$ was added, the temperature fell to 46°C-47°C. Within 5 minutes the exotherm started, the solution became thicker, and the $N_2$ flow was reduced and removed to the head space. The calculated heat of polymerization at room temperature was 22.5°C, based on a 25% aqueous acrylamide solution. External cooling was applied to maintain the temperature at or below 60°C. After completion of the exotherm, a temperature of 50°C was maintained. A sample was removed after 2 hours for acrylamide analysis, the nitrogen turned off, and 0.63 g of $NaHSO_3$ (0.5 mole % based on acrylamide) dissolved in 25 ml of $H_2O$ was added. After stirring for an hour, vacuum was pulled for 1-3 minutes several times over about a 15 minute period to help remove excess $SO_2$. While stirring vigorously, 118 g (0.059 m) of soyabis(polyoxyethylene)$_{15}$ amine was added with 75 ml of wash $H_2O$ over about 15 minutes period. After the additions, the pH was 8. Citric acid solution (25 g) was added to lower the pH to 6 +/- 0.5. The intrinsic viscosity of the polymer-sodium salt was 1.04 dl/g measured in 5.05 N NaCl at 29°C.

**Example 2 (76AM/25 AMPS)**

The 75AM/25AMPS™ copolymer was prepared by modifying the method described in U.S. Patent Nos. 4,578,267 by omitting the use of ethoxylated amine neutralizer.

A one liter resin kettle with overhead stirrer, $N_2$ inlet, condenser, thermocouple, heating mantle, and provision for external cooling was set up in a hood. DI water (250.25 g) along with 133.9 g of AMPS-sodium salt (50.5%, from Lubrizol) was charged to the kettle. Next, 378.95 g of 50% aqueous acrylamide from Cytec followed by 0.102 g of $CuCl_2$ dissolved in 155.25 g DI water was added and the mixture was heated to 50°C under $N_2$ with stirring. The heating mantle was removed and 1.3 g of $(NH_4)_2 S_2O_8$ dissolved in 62.5 ml of $H_2O$ was added. Within 2 minutes the exotherm started, the solution became thicker, and the $N_2$ flow was reduced and removed to the head space. The exotherm peaked at 99°C five minutes after the initiator addition. After completion of the exotherm, a temperature of 60°C was maintained for 3 hours with constant stirring. Samples were removed every hour for acrylamide and MW analysis. Sodium bisulfite (1.53 g dissolved in 90.25g DI water) was added. After stirring for an hour, vacuum was pulled for 1-3 minutes several times over about a 15 minute period to help remove excess $SO_2$. The reaction mixture was then cooled and packaged. It had a polymer solids content of 26.8% and pH 3.75. A viscosity of 122,000 centipoise (cps) was measured using a conventional Brookfield viscometer.

**Example 3 (Modified shot process, 90 AM/10 AMPS).**

The 90AM/10AMPS™ copolymer having improved performance was prepared by a modified shot process and provided a significantly shorter cycle time.

A one liter resin kettle with overhead stirrer, $N_2$ inlet, condenser, thermocouple, heating mantle, and provision for external cooling was set up in a hood. DI water (125 g) along with 25.43 g of AMPS-sodium salt (50.5%, from Lubrizol) was charged to the kettle. Next, 231.2 g of 50% aqueous acrylamide from Cytec followed by 0.051 g of $CuCl_2$ dissolved in 77.6 g DI water was added and the mixture was heated to 50°C under $N_2$ with stirring. The heating mantle was removed and 0.65 g of $(NH_4)_2 S_2O_8$ dissolved in 31 ml of $H_2O$ was added. Within 2 minutes the exotherm started, the solution became thicker, and the $N_2$ flow was reduced and removed to the head space. The exotherm peaked at 97°C four minutes after the initiator addition. After completion of the exotherm, a temperature of 85°C was maintained for 30 minutes with constant stirring. A sample was removed for acrylamide and MW analysis. Sodium bisulfite (0.77 g dissolved in 45 g DI water) was added. After stirring for an hour, vacuum was pulled for 1-3 minutes several times over about a 15 minute period to help remove excess $SO_2$. The reaction mixture was then cooled and packaged. It had a polymer solids content of 27.8%. A viscosity of >200,000 centipoise (cps) was measured using a conventional Brookfield viscometer.

**Example 4 (Modified shot process, 60 AM/40 AMPS)**

The 60AM/40AMPS™ copolymer having improved performance was prepared by a modified shot process and provided a significantly shorter cycle time.

A one liter resin kettle with overhead stirrer, $N_2$ inlet, condenser, thermocouple, heating mantle, and provision for external cooling was set up in a hood. DI water (125 g) along with 101.7 g of AMPS-sodium salt (50.5%, from Lubrizol) was charged to the kettle. Next, 154.2 g of 50% aqueous acrylamide from Cytec followed by 0.051 g of $CuCl_2$ dissolved in 77.6 g DI water was added and the mixture was heated to 50°C under $N_2$ with stirring. The heating mantle was removed and 0.65 g of $(NH_4)_2 S_2O_3$ dissolved in 31 ml of $H_2O$ was added. Within 2 minutes the exotherm started, the solution became thicker, and the $N_2$ flow was reduced and removed to the head space. The exotherm peaked at 92°C twenty-five minutes after the initiator addition. After completion of the exotherm, a temperature of 85°C was maintained for 30 minutes with constant stirring. A sample was removed for acrylamide and MW analysis. Sodium bisulfite (0.77 g dissolved in 45 g DI water) was added. After stirring for an hour, vacuum was pulled for 1-3 minutes several times over about a 15 minute period to help remove excess $SO_2$. The reaction mixture was then cooled and packaged. Polymer solids level was 26.9%. A viscosity of 45,100 centipoise (cps) was measured using a conventional Brookfield viscometer.

**Example 5 (Modified shot process, initiation at 40°C, 80 AM/20 AMPS).**

The 80AM/20AMPS™ copolymer having improved performance was prepared by a modified shot process and demonstrated that initiation can occur at lower temperatures, resulting in a lower, more controlled (safer) exotherm.

A one liter resin kettle with overhead stirrer, $N_2$ inlet, condenser, thermocouple, heating mantle, and provision for external cooling was set up in a hood. DI water (170 g) along with 51.4 g of AMPS-sodium salt (50.5%, from Lubrizol) was charged to the kettle. Next, 195.06 g of 50% aqueous acrylamide from Cytec followed by 0.051 g of $CuCl_2$ dissolved in 77.6 g DI water was added and the mixture was heated to 40°C under $N_2$ with stirring. The heating mantle was removed and 0.65 g of $(NH_4)_2 S_2O_8$ dissolved in 31 ml of $H_2O$ was added. Within 5 minutes the exotherm started, the solution became thicker, and the $N_2$ flow was reduced and removed to the head space. The exotherm peaked at 82°C fifteen minutes after the initiator addition. After completion of the exotherm, a temperature of 82°C was maintained for 60 minutes with constant stirring. Samples were periodically removed for acrylamide and MW analysis. The reaction mixture was then cooled and packaged. Polymer solids level was 25.6%. A viscosity of 167,000 centipoise (cps) was measured using a conventional Brookfield viscometer.

**Example 6 (Modified shot process, initiation at 40°C, 20 AM/80 AMPS).**

The 20AM/80AMPS™ copolymer having improved performance was prepared by a modified shot process and demonstrated that initiation can occur at lower temperatures, resulting in a lower, more controlled (safer) exotherm.

A one liter resin kettle with overhead stirrer, $N_2$ inlet, condenser, thermocouple, heating mantle, and provision for external cooling was set up in a hood. DI water (125 g) along with 205.6 g of AMPS-sodium salt (50.5%, from Lubrizol) was charged to the kettle. Next, 48.76 g of 50% aqueous acrylamide from Cytec followed by 0.051 g of $CuCl_2$ dissolved in 77.6 g DI water was added and the mixture was heated to 40°C under $N_2$ with stirring. The heating mantle was removed and 0.65 g of $(NH_4)_2 S_2O_8$ dissolved in 31 ml of $H_2O$ was added. Within 1 minute the exotherm started, the solution became thicker, and the $N_2$ flow was reduced and removed to the head space. The exotherm peaked at 65°C five minutes after the initiator addition. After completion of the exotherm, a temperature of 65°C was maintained for 120 minutes with constant stirring. Samples were periodically removed for acrylamide and MW analysis. The reaction mixture was then cooled and packaged. Polymer solids level was 30.0%. A viscosity of 4,900 centipoise (cps) was measured using a conventional Brookfield viscometer.

**Example 7 (Modified shot process, initiation at 50°C with low solids, 60 AM/40 AMPS).**

The 60AM/40AMPS™ copolymer having improved performance was prepared by a modified shot process and demonstrated that initiation can occur at lower solids, resulting in lower, more controlled (safer) exotherm and lower viscosity, both significant scale-up and manufacturing advantages.
A one liter resin kettle with overhead stirrer, $N_2$ inlet, condenser, thermocouple, heating mantle, and provision for external cooling was set up in a hood. DI water (165 g) along with 51.4 g of AMPS-sodium salt (50.5%, from Lubrizol) was charged to the kettle. Next, 72.32 g of 50% aqueous acrylamide from Cytec followed by 0.026 g of $CuCl_2$ dissolved in 77.6 g DI water was added and the mixture was heated to 50°C under $N_2$ with stirring. The heating mantle was removed and 0.325 g of $(NH_4)_2 S_2O_8$ dissolved in 31 ml of $H_2O$ was added. Within 1 minute the exotherm started, the solution became thicker, and the $N_2$ flow was reduced and removed to the head space. The exotherm peaked at 63°C fifteen minutes after the initiator addition. After completion of the exotherm, a temperature of 63°C was maintained for 30 minutes with constant stirring. Sodium bisulfite (0.28 g dissolved in 30 g DI water) was added. After stirring for 40 minutes at 63°C, the reaction mixture was then cooled and packaged. Polymer solids level was 14.5%. A viscosity of 1,670 centipoise (cps) was measured using a conventional Brookfield viscometer.

**Example 8 (Gradual Addition Process, 80 AM/20 AMPS).**

The 80AM/20AMPS™ copolymer having improved performance was prepared by a gradual addition process and resulted in a marked process improvement, in terms of temperature, viscosity and weight average molecular weight control.

A one liter resin kettle with overhead stirrer, $N_2$ inlet, condenser, thermocouple, heating mantle, and provision for external cooling was set up in a hood. DI water (170 g) was charged to the kettle and heated to 88°C under $N_2$. A mixture of 51.4 g of AMPS-sodium salt (50.5%, from Lubrizol) and 193.9 g of 50% aqueous acrylamide from Cytec was metered in to the reactor over 90 minutes along with a solution of 0.65 g of $(NH_4)_2 S_2O_8$ dissolved in 60 g of $H_2O$. The viscosity of the mixture gradually increased as the reaction proceeded. The mixture was diluted with 40 ml DI-water and held at 88°C for 30 minutes. It was then cooled to room temperature and packaged. Polymer solids level was 25.5% and viscosity was measured to be 7190 cps.

### Example 9 (Gradual Addition Process, 75 AM/25 AMPS)

The 75AM/25AMPS copolymer having improved performance was prepared by a gradual addition process.
Same as Example 8 but using 66.95 g AMPS and 179.69 g Am and 60 minute feed. 27.1% polymer solids and 13,650 cps viscosity were measured for the polymer.

### Example 10 (Gradual Addition Process, 60 AM/40 AMPS)

The 60AM/40AMPS™ copolymer having improved performance was prepared by a gradual addition process.
Same as Example 8 but using 102.8 g AMPS and 145.5 g Am. 25.4% polymer solids and 1420 cps viscosity were measured for the polymer.

### Example 11 (Gradual Addition Process, 40 AM/60 AMPS)

The 40AM/60AMPS™ copolymer having improved performance was prepared by a gradual addition process.
Same as Example 8 but using 154.2 g AMPS and 96.98 g Am. 26.4% polymer solids and 960 cps viscosity were measured for the polymer.

### Example 12 (Gradual Addition Process, 50 AM/50 AMPS)

The 50AM/50AMPS™ copolymer having improved performance was prepared by a gradual addition process.
Same as Example 8 but using 128.5 g AMPS and 121.22 g Am. 25.9% polymer solids and 630 cps viscosity were measured for the polymer.

### Example 13 (Gradual Addition Process, 20 AM/80 AMPS)

The 20AM/80AMPS™ copolymer having improved performance was prepared by a gradual addition process.
Same as Example 8 but using 205.6 g AMPS and 48.49 g Am. 25.7% polymer solids and 240 cps viscosity were measured for the polymer.

### Example 14 (Gradual Addition Process, 60 AM/40 AMPS-acid).

The 60AM/40AMPS™ copolymer having improved performance was prepared by a gradual addition process and demonstrates utility with solid grade (free acid form) of AMPS™ monomer.
A one liter resin kettle with overhead stirrer, $N_2$ inlet, condenser, thermocouple, heating mantle, and provision for external cooling was set up in a hood. DI water (120 g) was charged to the kettle and heated to 88°C under $N_2$. A mixture of 51.4 g of AMPS™ monomer (Lubrizol) dissolved 60 ml DI-water and 145.5 g of 53% aqueous acrylamide from Cytec was metered in to the reactor over 90 minutes along with a solution of 0.65 g of $(NH_4)_2 S_2O_8$ dissolved in 100 g of DI-$H_2O$. The viscosity of the mixture gradually increased as the reaction proceeded. Following addition of the reagents, the mixture was held at 88°C for 30 minutes and then a solution of 0.56 g sodium bisulfite dissolved in 40 g DI-$H_2O$ was added. The temperature was maintained at 88°C for another 30 minutes, then the mixture was cooled to room temperature and packaged. Polymer solids level 25.3%, pH 2.4 and a viscosity of 9320 cps were measured for the polymer.

### (Gradual Addition Process, 60 AM/40 AA). Comparative Example 2.

The 60AM/40AA copolymer having improved performance was prepared by a gradual addition process. The process exhibited poor polymerization kinetics, the polymer had high amounts of residual monomers and low formulation viscosity.
Same as Example 10 but with AA replacing AM.

### (Gradual Addition Process, 60 MAA/40 AMPS). Comparative Example 3.

The 60AM/40MAA copolymer having improved performance was prepared by a gradual addition process.
A one liter resin kettle with overhead stirrer, $N_2$ inlet, condenser, thermocouple, heating mantle, and provision for external cooling was set up in a hood. DI water (145 g) was charged to the kettle and heated to 88°C under $N_2$. A mixture of 75.4 g of AMPS-sodium salt (50.5%, from Lubrizol), 60.0 g of MAA (Rohm and Haas) and 20 g DI-$H_2O$ was metered in to the reactor over 90 minutes along with a solution of 0.50 g of $(NH_4)_2 S_2O_8$ dissolved in 20 g of $H_2O$ which was metered in over 97 minutes. The viscosity of the mixture gradually increased as the reaction proceeded. Following addition of the reagents, the mixture was held at 88°C for 5 minutes, then a solution of 0.25 g sodium bisulfite dissolved in 20 g DI-H$_2$O was metered in over 15 minutes. The mixture was cooled to room temperature and packaged.

**Example 15 (Gradual Addition Process, 60 AM/30 AMPS/10 AA).**

The 60AM/10AA/30AMPS™ terpolymer having improved performance was prepared by a gradual addition process.

A one liter resin kettle with overhead stirrer, N$_2$ inlet, condenser, thermocouple, heating mantle, and provision for external cooling was set up in a hood. DI water (170 g) was charged to the kettle and heated to 88°C under N$_2$. A mixture of 77.1 g of AMPS™-sodium salt (50.5%, from Lubrizol), 144.68 g of 53.3% aqueous acrylamide from Cytec, 12.85 g AA (Rohm and Haas) and 30 g DI-H$_2$O was metered in to the reactor over 90 minutes along with a solution of 0.65 g of (NH$_4$)$_2$S$_2$O$_8$ dissolved in 60 g of H$_2$O. The viscosity of the mixture gradually increased as the reaction proceeded. Following addition of the reagents, the mixture was held at 88°C for 30 minutes and then a solution of 0.56 g sodium bisulfite dissolved in 20 g DI-H$_2$O was added. The temperature was maintained at 88°C for another 30 minutes, then the mixture was cooled to room temperature and packaged. Polymer solids 27.2%, pH 4.4 and viscosity of 1240 cps were measured for the polymer.

**Example 16 (Gradual Addition Process, 60 AM/30 AMPS/10 MAA).**

The 60AM/10MAA/30AMPS™ terpolymer having improved performance was prepared by a gradual addition process.

Same as Example 15 except AA is replaced with MAA. Polymer solids level of 24.3%, pH 4.99 and viscosity of 840 cps were measured for the polymer.

**Example 17 (Gradual Addition Process, 75 AM/15 AMPS/10 MAA).**

The 75AM/10MAA/15AMPS terpolymer having improved performance was prepared by a gradual addition process.

Same as Example 15 but using 38.55 AMPS, 180.81 AM and 12.85 MAA. It had a polymer solids level of 26.9%, pH 4.69 and a viscosity of 3430 cPs was measured for the polymer.

**Example 18 (Gradual Addition Process, 60 AM/20 AMPS/20 MAA).**

The 60AM/20MAA/20AMPS™ terpolymer having improved performance was prepared by a gradual addition process.

Same as Example 15 but using 51.4 AMPS, 145.47 AM and 25.7 MAA and 15 g DI water rinse. Polymer solids 28.8%, pH 4.94 and a viscosity of 5200 cps were measured for the polymer.

**Example 19 (Gradual Addition Process, 60 AM/10 AMPS/30 MAA).**

The 60AM/30MAA/10AMPS™ terpolymer having improved performance was prepared by a gradual addition process.

Same as Example 15 but using 25.7 AMPS, 145.47 AM and 38.35 MAA. Polymer solids level of 28.4%, pH 4.94 and a viscosity of 3700 cps were measured for the polymer.

**Example 20 (Gradual Addition Process, 30 AM/60 AMPS/10 MAA).**

The 30AM/10MAA/60AMPS™ terpolymer having improved performance was prepared by a gradual addition process.

A one liter resin kettle with overhead stirrer, N$_2$ inlet, condenser, thermocouple, heating mantle, and provision for external cooling was set up in a hood. DI water (170 g) was charged to the kettle and heated to 88°C under N$_2$. A mixture of 154.2 g of AMPS™-sodium salt (50.5%, from Lubrizol), 72.73 g of 53.3% aqueous acrylamide from Cytec, 12.85 g MAA (Rohm and Haas) and 15 g DI-H$_2$O was metered in to the reactor over 90 minutes along with a solution of 0.65 g of (NH$_4$)$_2$S$_2$O$_8$ dissolved in 60 g of H$_2$O which was metered in over 95 minutes. The viscosity of the mixture gradually increased as the reaction proceeded. Following addition of the reagents, the mixture was held at 88°C for 10 minutes, then cooled to 60°C and a solution of 0.33 g sodium bisulfite dissolved in 20 g DI-H$_2$O was added. The temperature was maintained at 60°C for another 30 minutes, then the mixture was cooled to room temperature and packaged. It had a polymer solids level of 27.1%.

**Example 21 (Gradual Addition Process, 70 AM/25 AMPS/5 MAA).**

The 70AW5MAA/25AMPS™ terpolymer having improved performance was prepared by a gradual addition process.

Same as Example 20 but using 64.25 AMPS, 169.71 g AM, and 6.43 g MAA. It had a polymer solids level of 26.7%, pH 4.53 and a viscosity of 1540 cps was measured for the polymer.

**Example 22 (Gradual Addition Process, 75 AM/20 AMPS/5 MAA).**

The 75AM/5MAA/20AMPS™ terpolymer having improved performance was prepared by a gradual addition process.

Same as Example 20 but using 51.4 AMPS, 181.84 g AM, and 6.43 g MAA. Polymer solids of 26.6%, pH 4.58 and a viscosity of 2150 cps were measured for the polymer.

**Example 23 (Gradual Addition Process, 65 AM/32.5 AMPS/2.5 MAA).**

The 65AM/2.5MAA/32.5AMPS™ terpolymer having improved performance was prepared by a gradual addition process.

Same as Example 20 but using 83.52 AMPS, 157.59 g AM, and 3.21 g MAA. Polymer solids of 26.5%, pH 4.56 and a viscosity of 1360 cps were measured for the polymer.

**Example 24 (Gradual Addition Process, 60 AM/37.5 AMPS/2.5 MAA).**

The 60AM/2.5MAA/37.5AMPS™ terpolymer having improved performance was prepared by a gradual addition process.

Same as Example 20 but using 96.37 AMPS, 145.47 g AM, and 3.21 g MAA. Polymer solids level of 26.9%, pH 4.63 and a viscosity of 1300 cps were measured for the polymer.

**Example 25 (Gradual Addition Process, 65 AM/30 AMPS/5 MAA).**

The 65AM/5MAA/30AMPS™ terpolymer having improved performance was prepared by a gradual addition process.

A one liter resin kettle with overhead stirrer, $N_2$ inlet, condenser, thermocouple, heating mantle, and provision for external cooling was set up in a hood. DI water (170 g) was charged to the kettle and heated to 88°C under $N_2$. A mixture of 77.1 g of AMPS™-sodium salt (50.5%, from Lubrizol), 157.59 g of 53.3% aqueous acrylamide from Cytec, 6.43 g MAA (Rohm and Haas) and 15 g DI-$H_2$O was metered in to the reactor over 90 minutes along with a solution of 0.65 g of $(NH_4)_2 S_2O_8$ dissolved in 60 g of $H_2O$ which was metered in over 95 minutes. The viscosity of the mixture gradually increased as the reaction proceeded. Following addition of the reagents, the mixture was held at 88°C for 10 minutes, then cooled to 60°C and a solution of 0.33 g sodium bisulfite dissolved in 20 g DI-$H_2$O and 1.28g of 0.15% $FeSO_4 \cdot H_2O$ was added over 15 minutes. The temperature was maintained at 60°C for another 30 minutes, then the mixture was cooled to room temperature and packaged. Polymer solids level of 26.8%, pH 4.86 and a viscosity of 1440 cps were measured for the polymer.

**Example 26 (Gradual Addition Process, 65 AM/30 AMPS/5 MAA).**

The 65AM/5MAA/30AMPS™ terpolymer having improved performance was prepared by a gradual addition process.

A one liter resin kettle with overhead stirrer, $N_2$ inlet, condenser, thermocouple, heating mantle, and provision for external cooling was set up in a hood. DI water (145 g) was charged to the kettle and heated to 88°C under $N_2$. A mixture of 60 g of AMPS™-sodium salt (50.5%, from Lubrizol), 130 g of 53.3% aqueous acrylamide from Cytec, 5.0 g MAA (Rohm and Haas) and 20 g DI-$H_2$O was metered in to the reactor over 60 minutes along with a solution of 0.5 g of $(NH_4)_2 S_2O_8$ dissolved in 20 g of $H_2O$ which was metered in over 67 minutes. The viscosity of the mixture gradually increased as the reaction proceeded. Following addition of the reagents, the mixture was held at 88°C for 5 minutes, and a solution of 1.0 g of 0.15% $FeSO_4 \cdot H_2O$ was added. Next, 0.25 g sodium bisulfite dissolved in 10 g DI-$H_2$O and 0.25 g $(NH_4)_2 S_2O_8$ dissolved in 10 g of $H_2O$ was added over 15 minutes. The mixture was cooled to room temperature and packaged. Polymer solids of 26.4%, pH 4.4 and a viscosity of 1440 cps were measured for the polymer.

**Example 27 (Gradual Addition Process, 80 AM/17.5 AMPS/2.5 MAA).**

The 80AM/2.5MAA/17.5AMPS™ terpolymer having improved performance was prepared by a gradual addition process.

Same as Example 20 but using 47.54 AMPS, 193.97 g AM, and 3.21 g MAA. Polymer solids of 27.1%, pH 4.5 and a viscosity of 3760 cps were measured for the polymer.

**Example 28 (Gradual Addition Process, 60 AM/30 Ammonium AMPS/5 MAA).**

The 60AM/5MAA/30NH$_4$AMPS™ terpolymer having improved performance was prepared by a gradual addition process and demonstrates utility with AMPS™. ammonium salt.

A one liter resin kettle with overhead stirrer, N$_2$ inlet, condenser, thermocouple, heating mantle, and provision for external cooling was set up in a hood. DI water (145 g) was charged to the kettle and heated to 88°C under N$_2$. A mixture of 60.0 g of AMPS™-ammonium salt (50%, from Lubrizol), 130.0 g of AM (50% aqueous solution), 5 g of MAA (Rohm and Haas) and 20 g DI-H$_2$O was metered in to the reactor over 90 minutes along with a solution of 0.50 g of (NH$_4$)$_2$ S$_2$O$_8$ dissolved in 20 g of H$_2$O which was metered in over 97 minutes. The viscosity of the mixture gradually increased as the reaction proceeded. Following addition of the reagents, the mixture was held at 88°C for 5 minutes, then a solution of 0.25 g sodium bisulfite dissolved in 20 g DI-H$_2$O was metered in over 15 minutes. The mixture was cooled to room temperature and packaged.

**Hair Styling Formulations incorporating solution polymers 2-28**

Ingredients in a Typical Hair Styling Gel including the polymer used in the invention are summarized as follows:

| Ingredient | Wt. % |
|---|---|
| D.I. water | q.s to 100 |
| Alcohol | 0-90% A.I. |
| Viscosity Control Agent | 0.1-15% A.I. |
| Polymer used in the invention | 0.1-15% A.I. |
| Hair Fixative Polymer | 0-15% A.I. |
| Neutralizer | 0-15% A.I. |
| Humectant | 0-15% A.I. |
| Surfactant | 0-15% A.I. |
| Conditioning Agent | 0-15% A.I. |
| Silicone | 0-15% A.I. |
| Color | 0-15% A.I. |
| Fragrance | 0-15% A.I. |
| Preservatives | 0-15% A.I. |
| A. I. = active ingredient. | |

Suitable rheology modifiers include any thickeners and viscosity control agents listed under CTFA International Cosmetic Ingredients Dictionary and Handbook, including polycarboxylic acids, poly(meth)acrylic acids, vinyl polymers such as cross-linked acrylic acid polymers with the CTFA name Carbomer (Carbopol™ 940, 980, ETD™2020, Ultrez™ 10, Ultrez™ 21 available from Neveon), carboxylic acid/carboxylate copolymers such as acrylic acid/alkyl acrylate copolymers with the CTFA name Acrylates/C10-30 Alkyl acrylate cross polymer, cellulose derivates and modified cellulose polymers (Natrosol™ 250), (meth)acrylic alkoxyacrylate copolymers and (meth)acrylic acrylate copolymers (*e.g.* Aculyn™ 28, 33, 22), Guar gums, other gums, starch-based polymers, alginic acid-based polymers, and clays such as bentonite and laponite.

Suitable additional hair fixatives include any hair fixative polymers listed under CTFA International Cosmetic Ingredients Dictionary and Handbook, especially, PVP, PVP/VA Copolymer, Polyquaternium-4, Polyquaternium-11, PQ-7, PQ-39, PQ-2, PQ-10, PQ-16, PQ-16, PQ-46, PQ-28, PQ-55, ethyl ester of PVM/MA copolymers, butyl ester of PVM/MA copolymers, vinyl acetate/crotonic acid copolymers, VA/crotonates/vinyl neodecanoate copolymers, octylacrylamide/ acrylates butylaminoethyl methacrylate copolymers, PVP/dimethylaminoethyl methacrylate copolymers, Guar hydroxypropyl trimonium chloride, vinyl caprolactam/PVP/dimethyl aminoethyl methacrylate copolymers, PVP and dimethicones, PQ-28 and dimethicones, acrylates/hydroxyester-acrylates copolymers, PVP/vinylcaprolactam/DMAPA acrylate copolymers, PVP/DMAPA acrylate copolymers, Modified corn starch, polyvinylcaprolactam acrylate copolymers, isobutylene/ethylmaleimide/hydroxyethylmaleimide copolymers, alkylacrylates (C1-C20) and succinates/hydroxyacrylates copolymers (*e.g.* as Allianz™ LT-180).

Suitable neutralizers include any pH adjusters listed under CTFA International Cosmetic Ingredients Dictionary and Handbook, especially, Triethanolamine, Amino methyl propanil, Sodium hydroxide, Ammonium hydroxide, Potassium hydroxide, arginine, Tetrahydroxypropyl ethylenediamine, PEG-15 Cocamine, Diisopropanol amine, Triisopropanol amine, and combinations thereof.

Suitable humectants include any humectants listed under CTFA International Cosmetic Ingredients Dictionary and Handbook, especially glycerin, sorbitol, glycol, hydrolyzed wheat protein, polyethylene glycols (PEG), including PEG-4 to PEG-800, and PEG esters, and polyglyceryl sorbitol.

Suitable surfactants include any surfactants listed under CTFA International Cosmetic Ingredients Dictionary and Handbook having HLB 9-50, including, PPG-5-ceteth-20 (Proetyl AWS™, available from Croda), PEG-40 hydrogenated castor oil (Tagat™ CH 40, available from Goldschmidt), Oleth-20 (Brij 98™ available from Uniqema), Isoceteth-20 (Arlasolve™ 200 available from Uniqema), Nonoxynol-10 (Makon™ 10 available from Stepan), Polysorbate-20 (Tween™ 20 available from Uniqema).

Suitable conditioners include any hair conditioning agent listed under CTFA International Cosmetic Ingredients Dictionary and Handbook, especially, cationic conditioning quats, mono/di-alkyl quat (such as cetrimonium chloride, stearalkonium chloride, quaternium 82, benenyl trimethyl ammonium chloride, distearyl dimethyl ammonium chloride, hydrogenated tallow alkyl trimethyl ammonium chloride, dialkyl dimethyl ammonium chloride, ditallow alkyl dimethyl ammonium chloride, dicetyl dimethyl ammonium chloride, dibehenyl dimethyl ammonium chloride, stearyl dimethyl benzyl ammonium chloride, stearyl amidopropyl dimethyl ammonium benzyl chloride, ), polymeric quats (such as PQ-4, PQ-11, guar hydroxypropyltrimethylammonium chloride, PQ-43,44,52,53,55,55,56) , hydrolyzed wheat/soy/silk protein, elastin amino acids, lanolin alcohol, PEG-40 hydrogenated lanolin and panthenol.

Suitable silicones include volatile and non-volatile silicone conditioning agents, such as polydimethylsiloxane (known as dimethicone™), polydimethylsiloxane, polymethylphenylsiloxane, commercially available from GE Silicone, and Dow Corning; polyorganosiloxane materials, polyalkyleneoxide-modified siloxanes, amodimethicone amino-substituted siloxanes, and highly cross-linked polymeric siloxane systems, such as GE SS4230™ and SS4267™, commercially available dimethiconols™ and cyclomethicone™ (Dow Corning 1401™, 1402™, and 1403™ fluids), polymethylphenyl silicones, alkylated silicones, such as methyldecyl silicone and methyloctyl silicone, alkyl-modified siloxanes such as alkyl methicones and alkyl dimethicones with alkyl chains of C10-C50.

**Performance Evaluation Methods for Hair Gel Products/Resin Incorporating Solution Polymers (2-28) of Invention**

**1. High Humidity Curl Retention (HHCR)**

The curl retention properties of hair styling product/resin were measured at 25+2°C/90±2%RH over a period of time (up to 4 hr). The less changes in percentage curl retention vs. time is an indication of longer lasting hold performance of a hair styling product/resin.

Hair swatches were purchased from International Hair Importer, cut to 3.5 g and 8" (referred as initial $L_0$). The hair swatches were then washed by 10% stripping shampoo solution, comb through during rinsing with luke warm water. Excess water was squeezed out, then 0.5 g of sample product was applied to hair using a 1 cc syringe. Curled hair on a 5/8" curler, secure with metal hair pin. Dried hair on the curler in 45°C oven for 1hr, then continued drying at room temperature for 12 hour on a horizontal surface. To start the test, hair clips were removed, and hair slid off the curler. Hair curls were suspended on a plexiglass board. Initial reading of the curl heights ($L_i$) were measured. Curled hair samples were placed on a board located in a humidity and temperature control chamber. Curl heights were measured at specific time intervals ($L_t$). Percentage (%) curl retention is calculated as $L_0-L_t/L_0-L_i*100$

**2. Curl Stiffness and Stiffness Retention**

Curl stiffness and stiffness retention properties were measured at 77±2°F/50±2%RH. Good curl stiffness is an indication of crunchy curl and stiff hold, high stiffness retention values indicate durable hold.

Hair swatches were prepared in the same manor as in HHCR test. The hair swatches were then tested using a Diastron MTT160 Minature Tensile Tester-Cyclic Test Method. The hair swatches were compressed at 60mm/min, up to 66% compression for 5 cycles. The initial reading of the force of resistance at 1st compressing cycle is recorded as Curl Stiffness. The percent difference of resistance force between the 1st and 5th compressing cycle is calculated as Stiffness Retention.

**3. Hair Characteristics Evaluation**

Hair swatches were prepared follow the same guidelines of HHCR test method. Treated hair curls were suspended on a plexiglass board for panel evaluation based on the following performance aspects:

Dry Combability: Combed through the tress using large teeth of the comb, and evaluated force required to comb through tress. Less force required to comb through means better comb performance referred to as combability.
Hair Residue: After the hair is combed through, front and back of the curl is visually evaluated for flaking. Non-flaking tresses are rated at a value of 10.
Hair Feel: Evaluate feel and sensation of hair swatches between fingers. Silky feeling, soft hair feel translates to higher score value.

The scale is from 1 to 10. The higher the score, the better performance for that aspect.
The performance results for polymers of the invention as incorporated in a hair treatment formulation is summarized in Table 1.

Table 1. Testing data for invented polymers incorporated in hair formulations.

| SAMPLE | COMPOSITION | FORMULATED VISCOSITY | CLARITY FOR NTU | HHCR | STIFFNESS | Combability | Flaking | Halt Feel |
|---|---|---|---|---|---|---|---|---|
| 1 (COMP) | 75 AM/25 AMPS | | >50 | | | | | |
| 2* | 75 AM/25 AMPS | 17,300 | >20 | 92 | 238 | 7.0 | 8.2 | 5.3 |
| 3* | 90 AM/10 AMPS | 36,700 | >20 | 90 | 220 | 5.7 | 9 | 4 |
| 4* | 60 AM/40 AMPS | 12,450 | >10 | 90.9 | 221.7 | 6.7 | 8.8 | 5.3 |
| 5* | 80 AM/20 AMPS | 20,400 | >20 | 96 | 233.3 | 6.8 | 8.6 | 5.6 |
| 6* | 20 AM/80 AMPS | 10,200 | >10 | 91.4 | 273.3 | 6.6 | 7.8 | 5.8 |
| 7* | 60 AM/40 AMPS | 7,100 | >10 | | | | | |
| 8* | 80 AM/20 AMPS | 17,500 | >30 | | | | | |
| 9 | 75 AM/25 AMPS* | 11,950 | >20 | 89 | 190 | | | |
| 10 | 60 AM/40 AMPS* | 9,900 | >20 | | | | | |
| 11 | 40 AM/60 AMPS* | 7,800 | >20 | | | | | |
| 12 | 50 AM/50 AMPS* | 7,750 | >10 | | | | | |
| 13 | 20 AM/80 AMPS* | 10,600 | >10 | | | | | |
| 14 | 60 AM/40 AMPS (S)* | 11,750 | 8 | | | | | |
| 15 | 60 AM/30 AMPS/10 AA* | 8,000 | 5.5 | | | | | |
| 16 | 60 AM/30 AMPS/10 MAA* | 11,550 | 6.1 | | | | | |
| 17 | 75 AM/15 AMPS/10 MAA* | 13,700 | >10 | | | | | |
| 18 | 60 AM/20 AMPS/20 MAA* | 9,700 | 5.2 | | | | | |
| 19 | 60 AM/10 AMPS/30 MAA* | 10,950 | 5.2 | | | | | |
| 20 | 30 AM/60 AMPS/10 MAA* | 12,700 | 3.8 | 85 | 400 | 6.6 | 8.6 | 6.8 |
| 21 | 70 AM/25 AMPS/5 MAA | 16,650 | 7.3 | 85 | 320 | 7 | 8.2 | 6.8 |
| 22 | 75 AM/20 AMPS/5 MAA | 18,300 | 11.7 | | | | | |
| 23 | 65AM/32.5 AMPS/2.5 MAA | 18,150 | 7.3 | | | | | |
| 24 | 60 AM/37.5 AMPS/2.5 MAA | 15,200 | 5.3 | 85 | 372 | 6 | 8.4 | 7 |
| 25 | 65 AM/30 AMPS/5 MAA | 16,750 | 6.0 | 85 | 349 | 7.4 | 8.4 | 6.8 |

(continued)

| SAMPLE | COMPOSITION | FORMULATED VISCOSITY | CLARITY FOR NTU | HHCR | STIFFNESS | Combability | Flaking | Halt Feel |
|---|---|---|---|---|---|---|---|---|
| 26 | 65 AM/30 AMPS/5 MAA | 14,400 | 4.9 | | | | | |
| 27 | 80 AM/17.5 AMPS/2.5 MAA | 19,200 | >20 | | | | | |
| 28 | 65 AM/30 NH$_4$-AMPS/5 MAA | | | | | | | |
| COMP 2 | 60 Am/40 AA | 7,350 | 6.6 | | | | | |
| COMP 3 | 60 MAA/40 AMPS | | | | | | | |
| COMP 4 | MAA/AMPS | 10,750 | 6.1 | 70 | 304 | 7.7 | 9 | 6.3 |
| COMP 5 | MAA/MMA | 14,650 | 6.1 | 94 | 161 | 7.3 | 9.3 | 7.3 |

Comparative Example 4 is a MAA/AMPS™ copolymer known by the tradename Fixomer A-30™ and is available from Ondeo Nalco Company, Naperville, IL.

Comparative Example 5 is a MAA/methylmethacrylate MMA copolymer known by the tradename Fixate G-100™ and is available from Noveon.

NTU scale is a measure of formulation clarity. NTU values greater than 50 are not clear. NTU values greater than 30 are very hazy. NTU values greater than 20 are hazy. NTU values greater than 10 are slightly hazy. NTU values below 10 are visually and optically transparent (clear).

* outside the invention

AM/AMPS™ copolymers incorporated in the hair formulation (Examples 1-13) are not clear (Comparative Example 1) and range from very hazy (Example 8) to slightly hazy (Examples 4, 6, 7, 12 and 13). Addition of small amounts of acid-containing monomers including AA and MAA to AM/AMPS™ copolymers results also in unexpected formulation clarity (Examples 21-27). The multi-functional solution polymers in the hair fixative formulation used in the invention exhibit an excellent balance of water resistance versus water sensitivity. In addition, the invented polymers exhibit good color stability and good water stabilities and the polymers are non-flaking after application to hair. One advantage of these polymers is that the polymers are compatible when combined with additives including neutralizers, surfactants and thickeners. Moreover, addition of small of acid-containing monomers provides hair formulations that are compatible with thickeners such as carbomers, including Carbopol and result in hair formulations having stable formulation viscosities as shown in Table 1. Another advantage of these polymers is that the polymers can be neutralized at any stage of preparing the formulation.

The polymers used in the present invention have numerous advantages over polymers described in U. S. Pat. Nos. 6,569,413; 4,859,458; 4,578,267; and 4,401,650. All prior art publications describe AM/AMPS™ (also known as Lubrizol™) copolymers neutralized with basic compounds, including base addition salts and ethoxylated fatty amines. Polymers used in the invention differ significantly as compared with prior art copolymers in terms of their molecular weight distributions, polymer morphology, polymer properties and the process by which they are prepared. Polymers prepared according to and described in the latter three publications are good hair conditioners, but are poor hair fixatives. The compatibility of polymers used in the invention in thickeners including polycarboxylic acids, polyacrylic acids and carbomers including Carbopol™ is better than comparable anionic copolymers, such as MAA/AMPS™ and AM/AMPS™ copolymers. The % Curl retention (% CR) for the formulations comprising the invented polymers (Example 23, 74% CR) is also improved as compared to Comparative Examples 3 (67 % CR) and 4 (60 % CR).

## Claims

1. Use in a hair formulation of a multi-functional polymer comprising as monomer units: (a) 50 to 89.9 weight percent of acrylamide and (b) 10 to 40 weight percent of 2-acrylamido-2-methyl-1-propanesulfonic acid and salts thereof; wherein incorporating in the copolymer between 0.1 to 5 weight percent of one or more other acid-containing ethylenically unsaturated monomers provides clear formulations comprising the multi-functional polymer.

## Patentansprüche

1. Verwendung eines multifunlctionellen Polymers, umfassend als monomere Einheiten (a) 50 bis 89,9 Gew.-% Acrylamid und (b) 10 bis 40 Gew.-% 2-Acrylamido-2-methyl-1-propansulfonsäure und Salze davon, in einer Haarformulierung, wobei das Einmischen von zwischen 0,1 bis 5 Gew.-% von einem oder mehreren anderen säureenthaltenden, ethylenisch ungesättigten Monomer(en) in das Copolymer klare Formulierungen, welche das multifunktionelle Polymer umfassen, bereitstellt.

## Revendications

1. Utilisation dans une formulation capillaire d'un polymère multifonctionnel comprenant comme unités monomères : (a) 50 à 89,9 % en masse d'acrylamide et (b) 10 à 40 % en masse d'acide 2-acrylamido-2-méthyl-1-propanesulfonique et ses sels ; où l'incorporation dans le copolymère de 0,1 à 5 % en masse d'un ou plusieurs autres monomères éthyléniquement insaturés contenant des acides donne des formulations claires comprenant le polymère multifonctionnel.